# EUROPEAN PATENT SPECIFICATION

(11) **EP 3 972 530 B1**
(45) Date of publication and mention of the grant of the patent: **01.01.2025**
(21) Application number: 20729595.7
(22) Date of filing: 15.05.2020
(51) Int. Cl.: A61F 2/01, A61F 2/24

(54) **TAVR EMBOLIC PROTECTION VIA DEBRI CAPTURE OR DEFLECTION**
TAVR-EMBOLIESCHUTZ DURCH DAS ABFANGEN ODER ABLENKEN VON ABLAGERUNGEN
PROTECTION EMBOLIQUE DE TAVR PAR CAPTURE OU DÉVIATION DE DÉBRIS

(30) Priority: 20.05.2019 US 201962850103 P
(43) Date of publication of application: 30.03.2022
(73) Proprietor: St. Jude Medical, Cardiology Division, Inc., St. Paul, MN 55117 (US)
(72) Inventor: GALE, Caytlin, Minneapolis, MN 55413 (US); ERZBERGER, Gary, Plymouth, MN 55442 (US); MORIN, Kristen T., St. Paul, MN 55104 (US); SPRINGER, Trevor J., Stillwater, MN 55082 (US); OLSOE, Jaron J., Minneapolis, MN 55405 (US); VIETMEIER, Kristopher Henry, Monticello, MN 55362 (US); MORRISSEY, Michael Shane, St. Paul, MN 55116 (US); KLIMA, Daniel J., Andover, MN 55304 (US)
(74) Representative: Gill Jennings & Every LLP
(86) International application number: PCT/US2020/033030
(87) International publication number: WO 2020/236543

(56) References cited:
- WO-A1-2009/002548
- WO-A2-2011/132080
- CN-A- 103 349 577
- US-A1- 2016 058 540

## Description

### Background of the Disclosure

The present disclosure is related to protecting against embolism, and more particularly to devices, systems, and methods for the filtration and removal or deflection of debris within blood vessels.

Arterial embolism is a sudden interruption of blood flow to an organ or body part due to an embolus, *e.g.,* debris or a clot. During a surgical intervention, such as a cardiac intervention, a vascular intervention, or a coronary intervention, tissue, plaque, and/or other masses may be dislodged due to the intervention, resulting in an embolus. These emboli are capable of traveling far from their origins, migrating to other sites of the vasculature and resulting in potentially life threatening complications. For example, an embolus may travel through the carotid artery and inhibit the flow of blood to the brain, which may result in the death of brain cells, *i.e.,* cause a stroke. A blockage of the carotid arteries is the most common cause of a stroke.

One interventional procedure that may result in an increased risk of arterial embolism is transcatheter aortic valve replacement ("TAVR"). Prosthetic heart valves that are collapsible to a relatively small circumferential size can be delivered into a patient less invasively than valves that are not collapsible. For example, a collapsible valve may be delivered into a patient via a tube like delivery apparatus such as a catheter, a trocar, a laparoscopic instrument, or the like. This collapsibility can avoid the need for a more invasive procedure such as full open chest, open heart surgery.

Collapsible prosthetic heart valves typically take the form of a valve structure mounted on a stent. There are two types of stents on which the valve structures are ordinarily mounted: a self-expanding stent and a balloon expandable stent. To place such valves into a delivery apparatus and ultimately into a patient, the valve must first be collapsed or crimped to reduce its circumferential size.

When a collapsed prosthetic valve has reached the desired implant site in the patient (*e.g.,* at or near the annulus of the patient's heart valve that is to be replaced by the prosthetic valve), the prosthetic valve can be deployed or released from the delivery apparatus and re expanded to full operating size. For balloon expandable valves, this generally involves releasing the entire valve, assuring its proper location, and then expanding a balloon positioned within the valve stent. For self-expanding valves, on the other hand, the stent automatically expands as the sheath covering the valve is withdrawn.

In conventional delivery systems for self-expanding aortic valves, for example, after the delivery system has been positioned for deployment, the self-expanding prosthesis aortic valve may be released from an overlying sheath to allow the prosthetic aortic valve to expand into the native aortic valve to take over functioning of the native aortic valve.

During the deployment of the prosthetic heart valve, and in particular during the step of allowing the prosthetic heart valve to expand (or forcing the prosthetic heart valve to expand, for example via a balloon) into its final position in the native aortic valve, there is a risk that debris, such as plaque, may be dislodged from the anatomy and enter the flow of blood. In such scenarios, as noted above, it would be beneficial to be able to trap such debris and remove the debris from the body. However, even if such debris is not trapped and removed from the body, it may still be beneficial to deflect such debris so that the debris passes into the descending aorta. In other words, as described in greater detail below, if the debris cannot be trapped and removed from the body, it may still be a significant benefit to ensure that the debris avoids entering arteries branching from the ascending aorta.

US 2016/0058540 A1 discloses an apparatus and procedure for trapping embolic debris.

### Brief Summary of the Disclosure

According to the invention, a delivery device for a collapsible and expandable prosthetic heart valve includes an outer sheath extending from a proximal end to a distal end. A delivery sheath is positioned at the distal end of the outer sheath, the delivery sheath being sized and shaped to maintain the prosthetic heart valve in a collapsed condition therein. A filter sheath overlies a portion of the outer sheath. An intermediate sheath is positioned between the outer sheath and the filter sheath. An embolic filter has a proximal end coupled to the intermediate sheath, and a distal end that is not directly attached to the intermediate sheath. The embolic filter has a delivery condition in which the filter sheath overlies the embolic filter and the distal end of the embolic filter is in a collapsed condition, and a deployed condition in which the filter sheath does not overlie the embolic filter and the distal end of the embolic filter is in an expanded condition.

According to an aspect of the disclosure, a method of implanting a collapsible and expandable prosthetic aortic valve into a patient includes advancing a delivery device in the patient toward a native aortic valve annulus of the patient while the prosthetic aortic valve is maintained in a collapsed condition within a delivery sheath of the delivery device. Prior to deploying the prosthetic aortic valve, an embolic protection filter may be deployed from the delivery device into a position upstream of at least one ostium of an artery extending from an ascending aorta of the patient. The prosthetic aortic valve may be deployed into an expanded condition within the native aortic valve annulus while the embolic protection filter is deployed.

### Brief Description of the Drawings

Fig. 1 illustrates a schematic view of the aorta.
Fig. 2 is a side elevational view of a collapsible prosthetic heart valve in an expanded condition, showing the valve assembly attached to the stent.
Fig. 3A is side view of an operating handle for a transfemoral delivery device for a collapsible prosthetic heart valve, shown with a side elevational view of the distal portion of a transfemoral catheter assembly.
Figs. 3B-D are side and top views of the operating handle of Fig. 3A, and an enlarged side view of the hub in the proximal-most position, respectively.
Figs. 4A-B are highly schematic illustrations of an embolic protection device at different stages of deployment in a valve replacement procedure according to an aspect of the disclosure.
Figs. 5A-C are highly schematic illustrations of an embolic protection device at different stages of deployment in a valve replacement procedure according to another aspect of the disclosure.
Fig. 6A is a highly schematic illustration of a prosthetic heart valve delivery device with a deployed embolic protection device according to an aspect of the disclosure.
Fig. 6B is a perspective view of the delivery device of Fig. 6A.

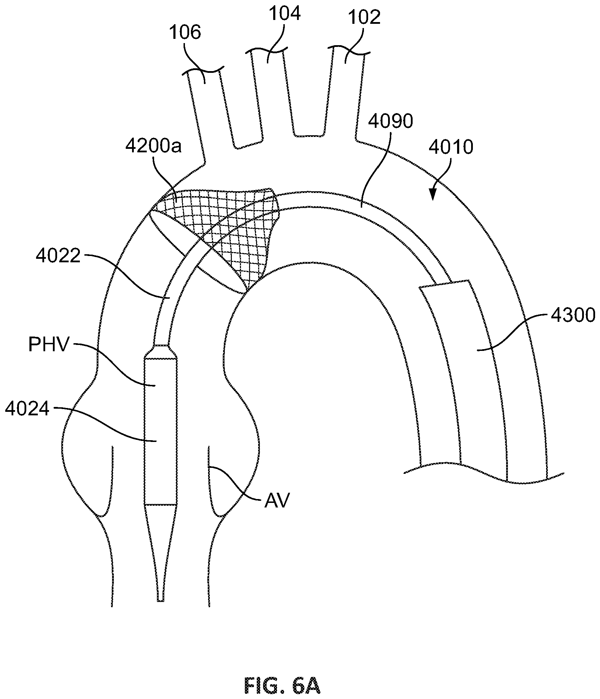

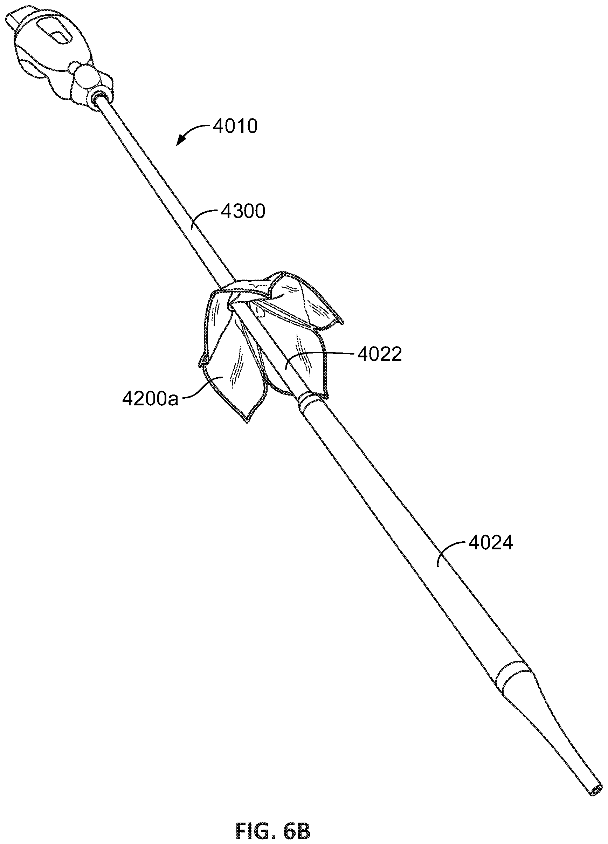

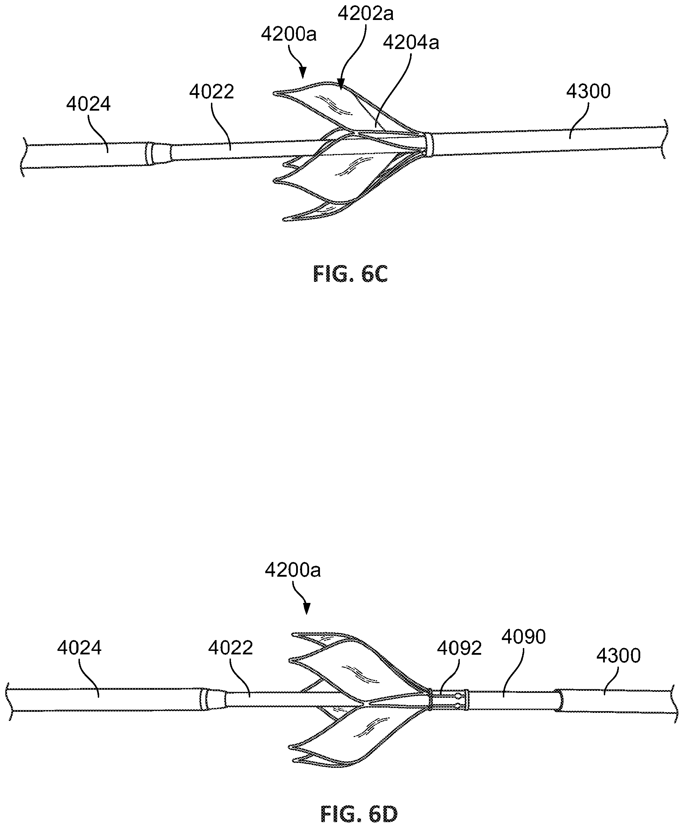

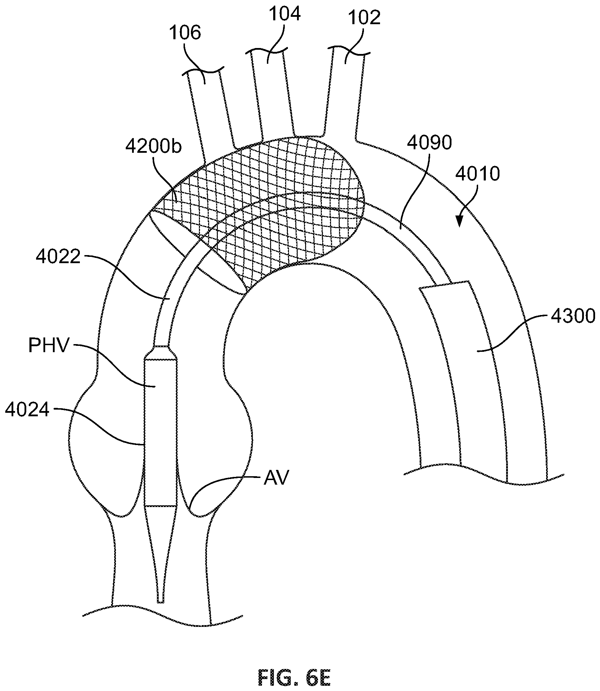

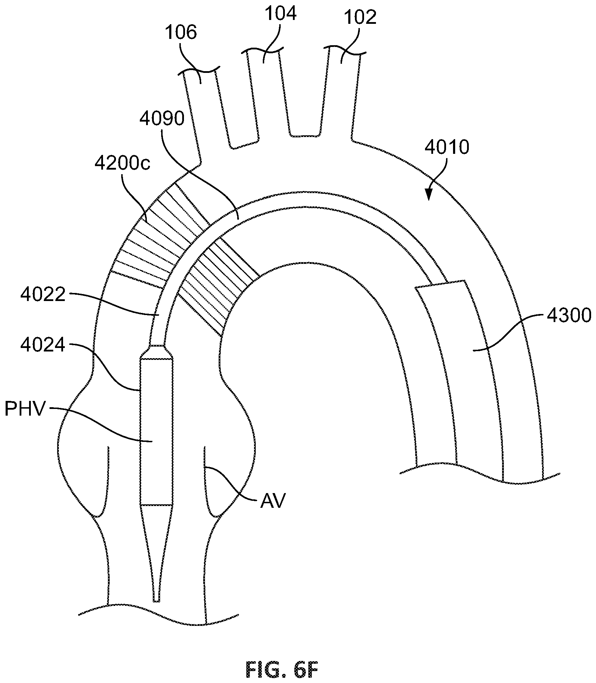

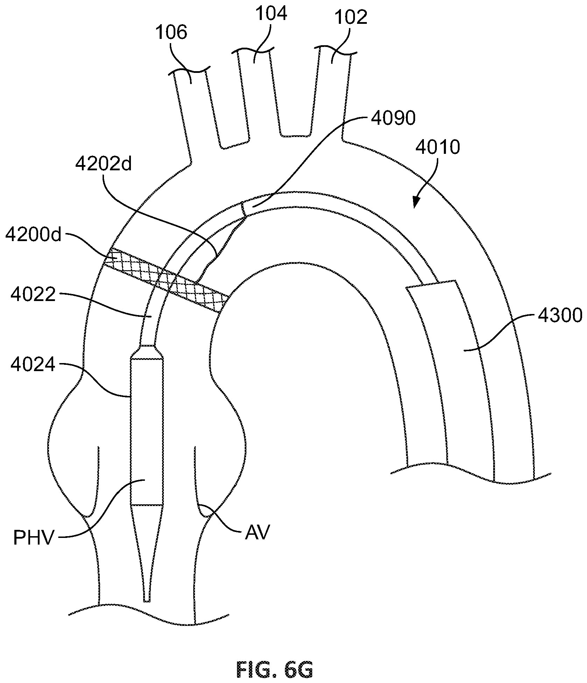

Figs. 6Cis a side view of the delivery device of Fig. 6B in a stage of deployment of the embolic protection device.
Fig. 6D illustrates the attachment of the embolic protection device to the delivery device of Fig. 6B.
Fig. 6E is a highly schematic illustration of the prosthetic heart valve delivery device of Fig. 6A with a first alternate embolic protection mechanism.
Fig. 6F is a highly schematic illustration of the prosthetic heart valve delivery device of Fig. 6A with a second alternate embolic protection mechanism.
Fig. 6G is a highly schematic illustration of the prosthetic heart valve delivery device of Fig. 6A with a second alternate embolic protection mechanism.

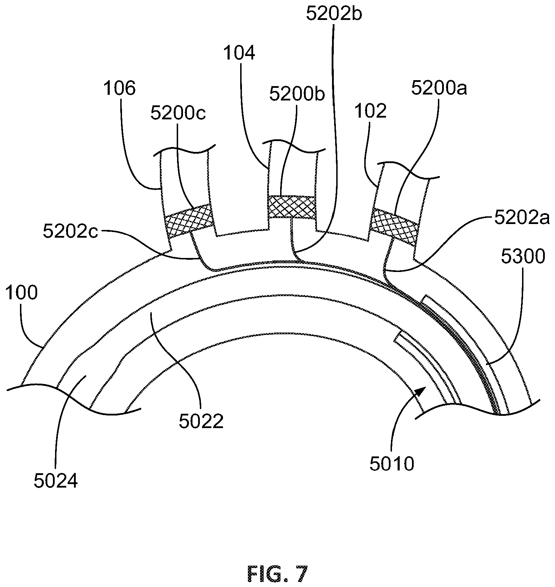

Fig. 7 is a highly schematic illustration of an embolic protection device deployed during a prosthetic heart valve replacement procedure according to another aspect of the disclosure.

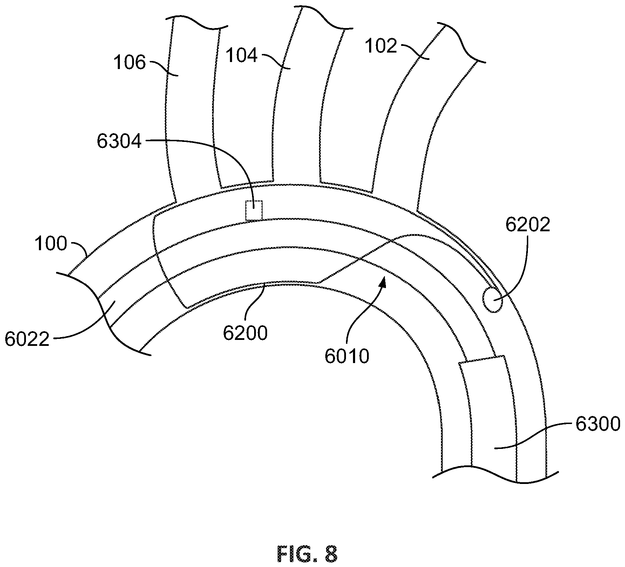

Fig. 8 is a highly schematic illustration of an embolic protection device deployed during a prosthetic heart valve replacement procedure according to a further aspect of the disclosure.

### Detailed Description

Particular embodiments of the present disclosure are described with reference to the accompanying drawings. In the figures and in the description that follow, like reference numerals identify similar or identical elements. As shown in the drawings and as described throughout the following description, when used in connection with a delivery device or associated components, the term "proximal" refers to the end of the device that is closer to the user and the term "distal" refers to the end of the device that is farther from the user.

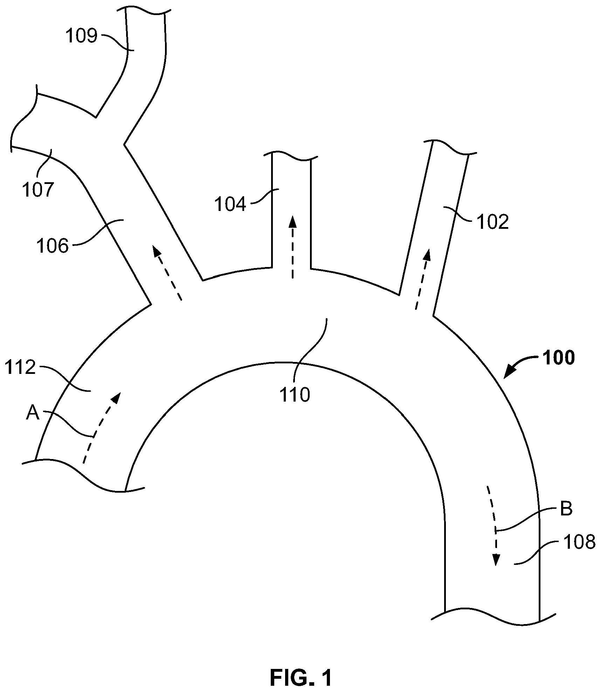

Fig. 1 illustrates aorta 100, the largest artery in the body, originating from the left ventricle (not shown) and extending down to the abdomen. Blood flows as indicated by arrow "A" from the left ventricle, through the aortic valve (not shown), through ascending aorta 112 to aortic arch 110. Three major arteries branch from aortic arch 110. Brachiocephalic artery 106 branches into right subclavian artery 107, supplying blood to the right arm, and right common carotid artery 109, supplying blood to the head and neck. Left common carotid artery 104 supplies blood to the head and neck. Left subclavian artery 102 supplies blood to the left arm. Blood from ascending aorta 112 not passing through one of these three arteries continues down descending aorta 108 as shown by arrow "B." Variations of the anatomy illustrated in Fig. 1 are possible and sometimes are relatively common. For example, about 10% of the population has a common brachiocephalic trunk, in which both common carotid arteries 104, 109 and right subclavian artery 107 arise from a single trunk off aortic arch 110. During interventional surgical, cardiac, and/or vascular procedures, there is a risk that emboli may break free and travel up ascending artery 112 and cause a blockage of brachiocephalic artery 106, right common carotid artery 109, and/or left common carotid artery 104, causing reduced blood flow to the brain and possibly a stroke. Devices and methods described herein may be used during interventional procedures to capture and remove emboli from the body. Further, as noted above, even if such emboli or debris are not able to be trapped and removed from the body, it may be a significant benefit to ensure that the debris is deflected from entering the brachiocephalic artery 106 and left common carotid artery 104, as such deflection may significantly reduce the risk of stroke or transient ischemic attach ("TIA").

Although so-called "embolic protection" devices exist in order to attempt to trap emboli during a cardiac intervention procedure, typically these devices are separate devices form the devices being used for the primary intervention, and as a result the use of a secondary embolic protection device may significantly increase both the complexity of the procedure and the length of the procedure. The devices described herein may mitigate increased complexity and duration that may otherwise occur when using an embolic protection device in conjunction with a TAVR procedure, at least partially due to the incorporation of the embolic protection features in the TAVR delivery device, described in greater detail below.

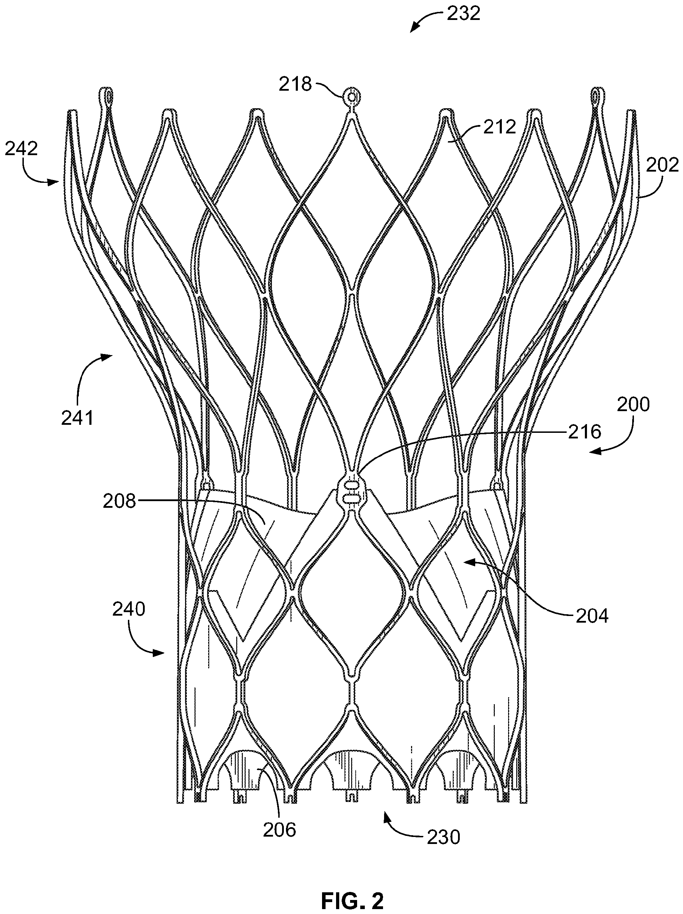

In order to assist in the understanding of TAVR valves and delivery devices, an exemplary valve and delivery device are briefly described. Fig. 2 shows a collapsible prosthetic heart valve 200. The prosthetic heart valve 200 is designed to replace the function of a native aortic valve of a patient. Examples of collapsible prosthetic heart valves are described in International Patent Application Publication No. WO/2009/042196; and United States Patent Nos. 7,018,406 and 7,329,278. The prosthetic heart valve has an expanded condition, shown in Fig. 2, and a collapsed condition.

Prosthetic heart valve 200 includes an expandable stent 202 which may be formed from any biocompatible material, such as metals, synthetic polymers or biopolymers capable of functioning as a stent. Stent 202 extends from a proximal or annulus end 230 to a distal or aortic end 232, and includes an annulus section 240 adjacent the proximal end and an aortic section 242 adjacent the distal end. The annulus section 240 has a relatively small cross section in the expanded condition, while the aortic section 242 has a relatively large cross section in the expanded condition. Preferably, annulus section 240 is in the form of a cylinder having a substantially constant diameter along its length. A transition section 241 may taper outwardly from the annulus section 240 to the aortic section 242. Each of the sections of the stent 202 includes a plurality of cells 212 connected to one another in one or more annular rows around the stent. For example, as shown in Fig. 2, the annulus section 240 may have two annular rows of complete cells 212 and the aortic section 242 and transition section 241 may each have one or more annular rows of partial cells 212. The cells 212 in the aortic section 242 may be larger than the cells 212 in the annulus section 240. The larger cells in the aortic section 242 better enable the prosthetic valve 200 to be positioned without the stent structure interfering with blood flow to the coronary arteries.

Stent 202 may include one or more retaining elements 218 at the distal end 232 thereof, the retaining elements being sized and shaped to cooperate with female retaining structures provided on the deployment device. The engagement of retaining elements 218 with the female retaining structures on the deployment device helps maintain prosthetic heart valve 200 in assembled relationship with the deployment device, minimizes longitudinal movement of the prosthetic heart valve relative to the deployment device during unsheathing or resheathing procedures, and helps prevent rotation of the prosthetic heart valve relative to the deployment device as the deployment device is advanced to the target location and during deployment.

The prosthetic heart valve 200 includes a valve assembly 204 positioned in the annulus section 240. Valve assembly 204 includes a cuff 206 and a plurality of leaflets 208 which collectively function as a one way valve. The commissure between adjacent leaflets 208 may be connected to commissure features 216 on stent 202. Fig. 2 illustrates a prosthetic heart valve for replacing a native tricuspid valve, such as the aortic valve. Accordingly, prosthetic heart valve 200 is shown in Fig. 2 with three leaflets 208, as well as three commissure features 216. As can be seen in Fig. 2, the commissure features 216 may lie at the intersection of four cells 212, two of the cells being adjacent one another in the same annular row, and the other two cells being in different annular rows and lying in end to end relationship. Preferably, commissure features 216 are positioned entirely within annulus section 240 or at the juncture of annulus section 240 and transition section 241. Commissure features 216 may include one or more eyelets which facilitate the suturing of the leaflet commissure to the stent. However, it will be appreciated that the prosthetic heart valves may have a greater or lesser number of leaflets and commissure features. Additionally, although cuff 206 is shown in Fig. 1 as being disposed on the luminal surface of annulus section 240, it is contemplated that the cuff may be disposed on the abluminal surface of annulus section 240, or may cover all or part of either or both of the luminal and abluminal surfaces of annulus section 240. Both the cuff 206 and the leaflets 208 may be wholly or partly formed of any suitable biological material or polymer.

In operation, a prosthetic heart valve, including the prosthetic heart valve described above, may be used to replace a native heart valve, such as the aortic valve, a surgical heart valve or a heart valve that has undergone a surgical procedure. The prosthetic heart valve may be delivered to the desired site (*e.g.,* near a native aortic annulus) using any suitable delivery device, including the delivery devices described below. During delivery, the prosthetic heart valve is disposed inside the delivery device in the collapsed condition. The delivery device may be introduced into a patient using a transfemoral, transapical or transseptal approach. Once the delivery device has reached the target site, the user may deploy the prosthetic heart valve. Upon deployment, the prosthetic heart valve expands into secure engagement within the native aortic annulus. When the prosthetic heart valve is properly positioned inside the heart, it works as a one-way valve, allowing blood to flow in one direction and preventing blood from flowing in the opposite direction.

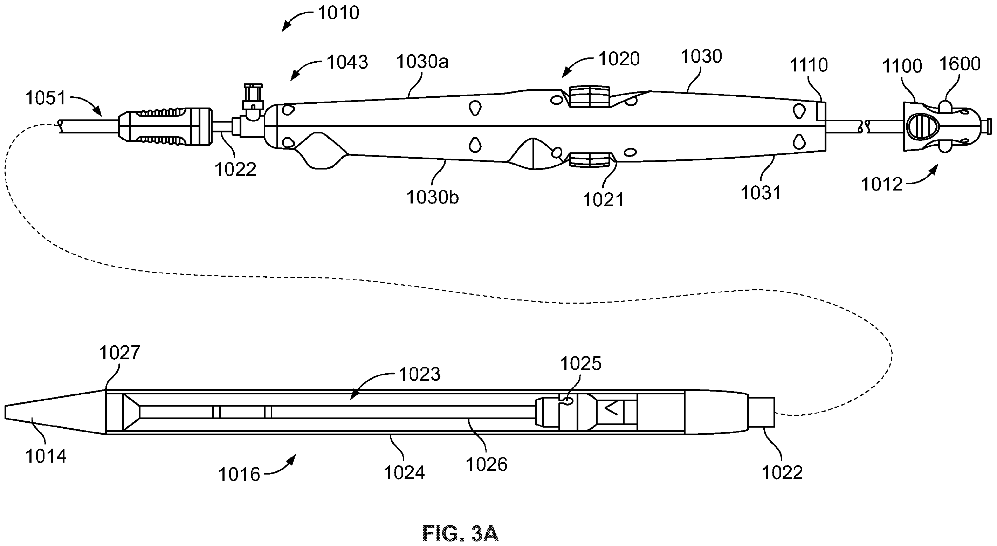

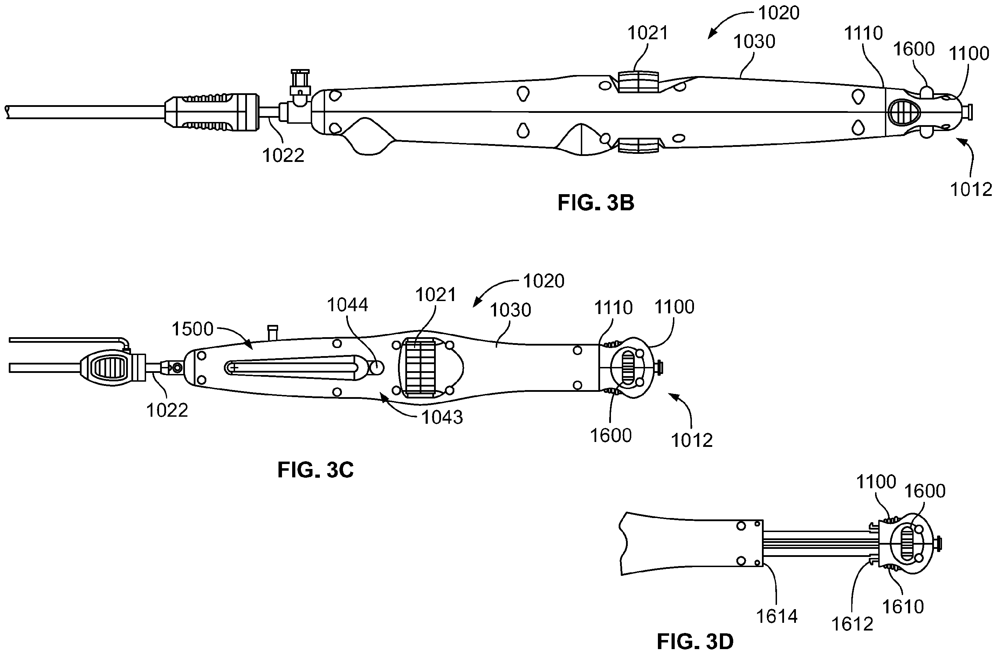

Turning now to Figs. 3A-D, an exemplary transfemoral delivery device 1010 for a collapsible prosthetic heart valve (or other types of self-expanding collapsible stents) has a catheter assembly 1016 for delivering the heart valve to and deploying the heart valve at a target location, and an operating handle 1020 for controlling deployment of the valve from the catheter assembly. The delivery device 1010 extends from a proximal end 1012 to a distal tip 1014. The catheter assembly 1016 is adapted to receive a collapsible prosthetic heart valve (not shown) in a compartment 1023 defined around an inner shaft 1026 and covered by a distal sheath 1024. The inner shaft 1026 extends through the operating handle 1020 to the distal tip 1014 of the delivery device, and includes a retainer 1025 affixed thereto at a spaced distance from distal tip 1014 and adapted to hold a collapsible prosthetic valve in the compartment 1023.

The distal sheath 1024 surrounds the inner shaft 1026 and is slidable relative to the inner shaft such that it can selectively cover or uncover the compartment 1023. The distal sheath 1024 is affixed at its proximal end to an outer shaft 1022, the proximal end of which is connected to the operating handle 1020. The distal end 1027 of the distal sheath 1024 abuts the distal tip 1014 when the distal sheath fully covers the compartment 1023, and is spaced apart from the distal tip 1014 when the compartment 1023 is at least partially uncovered.

The operating handle 1020 is adapted to control deployment of a prosthetic valve located in the compartment 1023 by permitting a user to selectively slide the outer shaft 1022 proximally or distally relative to the inner shaft 1026, or to slide the inner shaft 1026 relative to the outer shaft 1022, thereby respectively uncovering or covering the compartment with the distal sheath 1024. Operating handle 1020 includes frame 1030 which extends from a proximal end 1031 to a distal end and includes a top frame portion 1030a and a bottom frame portion 1030b. The proximal end of the inner shaft 1026 is coupled to a hub 1100, and the proximal end of the outer shaft 1022 is affixed to a carriage assembly that is slidable within the operating handle along a longitudinal axis of the frame 1030, such that a user can selectively slide the outer shaft relative to the inner shaft by sliding the carriage assembly relative to the frame. Alternatively, inner shaft 1026 may be actuated via hub 1100 to cover or uncover the compartment. Optionally, an introducer sheath 1051 is disposed over some or all of outer shaft 1022. The introducer sheath 1051 may be attached to the outer shaft 1022 or may be unattached. Additionally, introducer sheath 1051 may be disposed over a majority of outer shaft 1022 or over a minority of the outer shaft (e.g., over 49% or less, over 33%, etc.). Further, a stability layer may be provided between outer shaft 1022 and introducer sheath 1051. The stability layer may be translationally fixed to a distal end portion of operating handle 1020, and may extend any desired length distally toward distal sheath 1024. The stability layer may optionally be more rigid than the outer shaft 1022. The stability layer may also be referred to as a stability tube, stability sheath, or an intermediate sheath herein.

Additionally, hub 1100 may include a pair of buttons 1610, each attached to a clip 1612 (Fig. 3D). Clips 1612 on hub 1100 may mate with voids 1614 on frame 1030 to ensure that the hub and the frame are securely coupled together. Optionally, hub 1100 may also include a wheel 1600, which will be described in greater detail below.

A first mechanism for covering and uncovering the compartment 1023 will be referred to as a "fine" technique as covering and uncovering occurs slowly with a high degree of precision. To allow for this technique, frame 1030 defines an elongated space in which the carriage assembly may travel. The elongated space preferably permits the carriage assembly to travel a distance that is at least as long as the anticipated length of the prosthetic valve to be delivered (*e.g.,* at least about 50 mm), such that the distal sheath 1024 can be fully retracted off of the prosthetic valve.

The carriage assembly includes a main body and a threaded rod extending proximally therefrom along the longitudinal axis of the frame 1030. The threaded rod preferably is longer than the anticipated maximum travel distance of the carriage assembly within the elongated space (*e.g.,* at least about 50 mm), such that the threaded rod does not fully withdraw from the elongated space during deployment of the prosthetic valve.

A deployment actuator 1021, shown in Figs. 3A-D as a wheel protruding from the upper and lower frames 1030a, 1030b is fixedly coupled to a first gear so that rotation of actuator 1021 causes a corresponding rotation of the first gear. The first gear, in turn, is threadedly engaged on the threaded rod of the carriage. The first gear converts rotation of deployment actuator 1021 into longitudinal translation of the threaded rod of the carriage and a corresponding translation of the main body of the carriage. Hence, rotation of actuator 1021 in one direction (either clockwise or counterclockwise depending on the orientation of the threads on the threaded rod) causes the carriage assembly to translate proximally within the elongated space. Alternatively, the actuator 1021 and the first gear may be integral with one another.

As outer shaft 1022 is fixedly connected to the carriage assembly, translation of the carriage assembly results in a longitudinal translation of outer shaft 1022 and with it distal sheath 1024. Thus, deployment actuator 1021 is configured to provide for fine movement of outer shaft 1022 for deployment and recapture of the prosthetic heart valve. As deployment actuator 1021 protrudes from upper and lower frames 1030a, 1030b approximately halfway between the proximal and distal ends of the handle 1020, a user may readily rotate the actuator with his or her thumb and/or index finger.

Optionally, handle 1020 further includes a resheathing lock 1043 adapted to prevent any movement of the carriage assembly within the frame 1030, thereby preventing a user from accidentally initiating deployment of a prosthetic valve (Fig. 3C). Resheathing lock 1043 may be coupled to the main body of the carriage assembly 1040. The resheathing lock 1043 may include a laterally projecting pin 1044 that is slidable within a hole in the main body of the carriage assembly. Pin 1044 may have a first or unlocked condition in which it is compressed between main body 1041 and frame 1030.

As the user rotates deployment actuator 1021, outer shaft 1022 is pulled back and with it distal sheath 1024 to uncover a portion of compartment 1023. This process may continue until a predetermined position just prior to a position at which resheathing is no longer possible. When this predetermined position is reached, a spring positioned in the hole between the main body of the carriage assembly and the pin 1044 pushes the pin out through an aperture in frame 1030 to a second or locked condition in which the pin protrudes from frame 1030, providing a visual indicator to the user that resheathing is no longer possible past this predetermined position. Further translation of the carriage assembly may be impeded until the user presses pin 1044 to the interior of frame 1030 against the action of spring 1045 to confirm that further uncovering of compartment 1023 is desired (i.e., that the user wishes to fully deploy the prosthetic heart valve in its current position).

The initial distance that the carriage assembly can travel before actuating resheathing lock 1043 may depend on the structure and size of the particular prosthetic valve to be deployed. Preferably, the initial travel distance of the carriage assembly is about 3 mm to about 5 mm less than the length of the valve in the collapsed condition (*e.g.,* about 3 mm to about 5 mm of the valve may remain covered to permit resheathing). Alternatively, the initial travel distance of the carriage assembly may be about 40 mm to about 45 mm, which is about 80% to about 90% of the length of an exemplary 50 mm valve. Thus, resheathing lock 1043 may allow uncovering of compartment 1023 up to a maximum distance or percentage, and allow further uncovering only after the user has pressed on laterally projecting pin 1044 to confirm that additional release (*e.g.,* full release of the prosthetic heart valve) is desired.

A second technique, referred to as a "coarse technique," may be used to cover and uncover compartment 1023 more quickly and with less precision than the fine technique described above. Specifically, hub 1100 may be coupled to the proximal end of inner shaft 1026 and may be capable of moving the inner shaft relative to frame 1030 to facilitate opening and closing of the compartment 1023. This coarse movement may be used when no prosthetic heart valve is present in the compartment, such as, for example, when the compartment is to be opened prior to loading the prosthetic heart valve, and when the compartment is to be closed after the valve has been fully deployed. A mechanical lock 1110 may couple hub 1100 to frame 1030 to prevent accidental movement during use of operating handle 1020. For example, hub 1100 and a portion of frame 1030 may be threadedly engaged such that a rotation of the hub relative to the frame is required to release the hub from the frame. Other types of mechanical locks that will releasably couple hub 1100 to frame 1030 as intended will be known to those skilled in the art. After lock 1110 has been disengaged, hub 1100 may be used to quickly cover or uncover compartment 1023. Movement of inner shaft 1026 with respect to outer shaft 1022 may open and close the compartment. Thus, pushing hub 1100 distally (and thus the distal movement of inner shaft 1022) opens compartment 1023 and pulling hub 1100 proximally closes the compartment.

Optionally, an indicator window 1500 may be disposed on top of frame 1030 and may include a series of increments showing a percent or extent of deployment of the prosthetic heart valve. A scrolling bar may move along window 1500 past the series of increments as deployment continues to illustrate to the user the extent to which the prosthetic heart valve has been deployed. Scrolling bar indicates that a prosthetic heart valve is approximately 37.5% deployed. Indicator window 1500 further includes a critical indicator showing the position past which resheathing is no longer possible. Resheathing lock 1043 may be activated as the scrolling bar, which is coupled to the main body of the carriage assembly reaches position the critical indicator position. Additional features of delivery devices that may be suitable for use in delivering a prosthetic valve similar to prosthetic valve 200 are described in U.S. Patent Publication No. 2018/0325669.

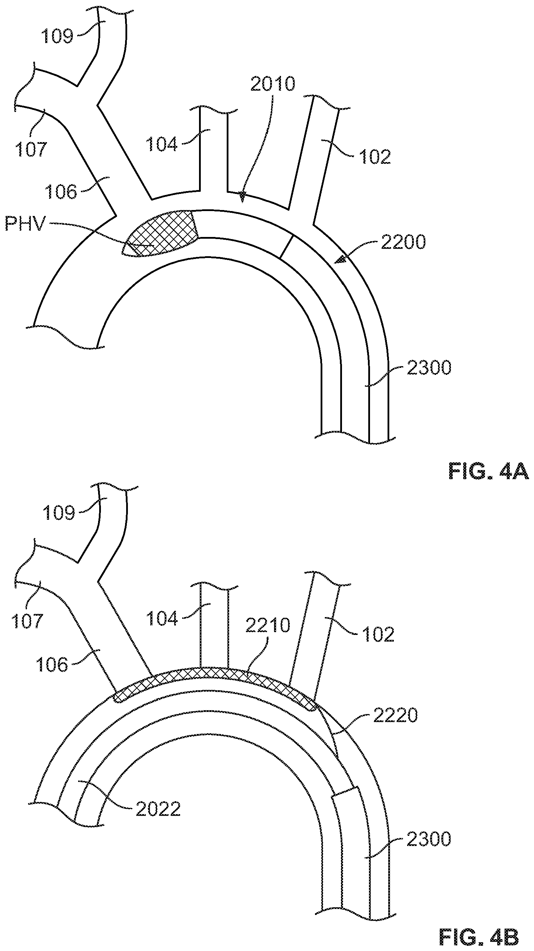

As noted above, although embolic protection devices have been used in interventional procedures, typically the use of such devices during a TAVR procedure increase complexity and the time required for the procedure. Figs. 4A-B illustrate a delivery device 2010 that includes embolic protection features according to an aspect of the disclosure. Referring to Fig. 4A, a delivery device 2010 is illustrated passing through the aortic arch as a collapsed prosthetic heart valve PHV is being advanced toward the native aortic valve. Prosthetic heart valve PHV may be any suitable collapsible and expandable prosthetic aortic heart valve, such as prosthetic heart valve 200. Delivery device 2010 may be substantially similar or identical to delivery device 1010, with certain modifications described below. However, it should be understood that the modifications may be applied to other prosthetic heart valve delivery devices that are not identical to delivery device 1010.

In use, the prosthetic heart valve PHV, held in a collapsed condition within delivery device 2010, is advanced to the native aortic valve annulus. Prior to deploying the prosthetic heart valve PHV, a filter 2200 may be deployed from the delivery device 2010 to cover the ostia of one or more of the arteries 102, 104, 106 extending from the aorta. In Fig. 4A, the filter 2200 is illustrated as being maintained in a collapsed delivery condition by an overlying filter sheath 2300. When the prosthetic heart valve PHV is in the desired deployment position, but still maintained in a collapsed condition, filter sheath 2300 may be retracted to allow filter 2200 to transition to a deployed condition, as shown in Fig. 4B. Filter 2200 may include a filter section 2210 and a connection member 2220. The filter section 2210 may be formed of a shape memory metal, including for example nitinol. The filter section 2210 may be formed of a single component or multiple components. For example, the filter section 2210 may be formed of a scaffold that includes a fabric or other material mounted to the scaffold. Otherwise, the filter section 2210 may be formed of a single material, such as nitinol, braided together into a desired shape. However, it should be understood that various other metal and non-metal materials may be suitable to form filter section 2210. Regardless of the material forming the filter section 2210, or the particular structure of the filter section 2210, the filter section 2210 preferably is porous enough to allow blood to flow through the filter section 2210, but not so porous as to allow debris, such as emboli, to pass through the filter section 2210. Thus, for example, if filter section 2210 is formed of braided nitinol, the density of the braid should be low enough to allow blood to pass through the braid, but high enough to capture or deflect debris such as emboli. The filter section 2210 may have any suitable shape that allows it to be compressed for delivery, and to cover the desired ostia of the arteries extending from the aorta. For example, as shown in Fig. 4B, the filter section 2210 may be long and flat, such as having an elongated oval shape, so that in the deployed condition, the filter section covers the ostium of each artery 102, 104, 106 in the deployed condition. It should be understood that other shapes may be suitable. In addition, rather than being flat, the filter section 2210 may include an amount of curvature to better conform to the curvature of the wall(s) of the aorta.

A proximal end of the filter section 2210 may be coupled to the delivery device 2010 via connection member 2220. In the illustrated embodiment, connection member 2220 preferably takes the form of a thin arm, and preferably has enough rigidity to help the filter section 2210 maintain its position relative to the point of connection to the delivery device 2010 when deployed. In addition, an amount of rigidity of the connection member 2220 may assist in guiding the filter section 2210 back into a collapsed condition within the filter sheath 2300 prior to removal of the delivery device 2010 upon completion of the procedure. In some embodiments, connection member 2220 may be a single metallic wire such as a nitinol wire, or a group of wires. The distal end of the connection member 2220 may be integral with, or otherwise attached to, the filter section 2210. The proximal end of the connection member 2220 may be coupled to any component of the delivery device 2010 that is capable of maintaining a substantially static position relative to the aorta 100 while the outer sheath 2022 is retracted for deployment of the prosthetic heart valve PHV. In the illustrated embodiment, the proximal end of the connection member 2220 may be fixed to a portion of a stability layer, which may be similar or identical to the stability layer described in connection with delivery device 1010. Although the stability layer is not separately labeled in Fig.4B, the stability layer is translationally fixed to a distal end portion of an operating handle, similar to operating handle 1010, and directly overlies the outer sheath 2022. The stability layer may extend distally from the operating handle to a location that is spaced proximally from the distal sheath that houses the prosthetic heart valve PHV, so that the outer sheath 2022 may be retracted within stability layer to fully deploy the prosthetic heart valve PHV. However, in other embodiments, the proximal end of the connection member 2220 may be coupled to other components of the delivery device 2010, such as by extending proximally, interior to filter sheath 2300, to the operating handle or other component.

With the prosthetic heart valve PHV in the collapsed condition and positioned within or adjacent the native aortic valve, filter sheath 2300 may be retracted to deploy filter 2200. Filter sheath 2300 may be similar or the same as introducer sheath 1051 described in connection with delivery device 1010, or may be a separate sheath positioned outside of outer sheath 2022 and inside of an introducer sheath similar to introducer sheath 1051, if such an introducer sheath is included in delivery device 2010. As the filter sheath 2300 is retracted, the filter section 2210 may begin to expand or otherwise transition from a delivery state (shown in Fig. 4A) to a deployed state (shown in Fig. 4B) in which the filter section 2210 covers the ostia of one or more of the arteries 102, 104, 106 extending from the aorta. As the filter sheath 2300 retracts so that it no longer overlies connection member 2220, the connection member 2220 may help the filter section 2210 spring, pop, or otherwise transition into the desired position. With the filter 2220 deployed in the desired position, as shown in Fig. 4B, the outer sheath 2022 may be retracted to deploy the prosthetic heart valve PHV into the native aortic valve annulus. The retraction of the outer sheath 2022 does not substantially affect the position of the filter 2200 relative to the ostia of the arteries 102, 104, 106, at least because the connection member 2220 is coupled to a component that does not translate as a result of retraction of the outer sheath 2022. During deployment of the prosthetic heart valve PHV, any tissue or other debris that may be dislodged and travel in the bloodstream through the aorta 100 is likely to be either trapped within the filter portion 2210, or deflected into the descending aorta, reducing the risk of stroke or TIA. Once the prosthetic heart valve PHV is implanted in a desired manner, the outer sheath 2022 may be advanced to close the compartment that previously housed the prosthetic heart valve PHV. Alternatively, a hub similar to hub 1100 of delivery device 1010 may be pulled proximally to result in a "coarse" movement to close the capsule after the prosthetic heart valve PHV is fully deployed. Then, the filter sheath 2300 may be advanced distally relative to the outer sheath 2022 and/or the stability layer. The filter sheath 2300 will ride along the connection member 2220, forcing the connection member to a position between the filter sheath 2300 and the outer sheath 2022 and/or the stability layer. As advancement of the filter sheath 2300 continues, the filter portion 2210, along with any debris trapped therein, is also forced back into the collapsed condition within the filter sheath 2300, similar to the condition shown in Fig. 4A. Then, the delivery system 2010 may be removed from the body, completing the procedure. As should be understood, compared to the use of a separate embolic protection device, the provision of the filter 2200 on the delivery device 2010 that also contains the prosthetic heart valve PHV may significantly reduce the complexity and time of the procedure.

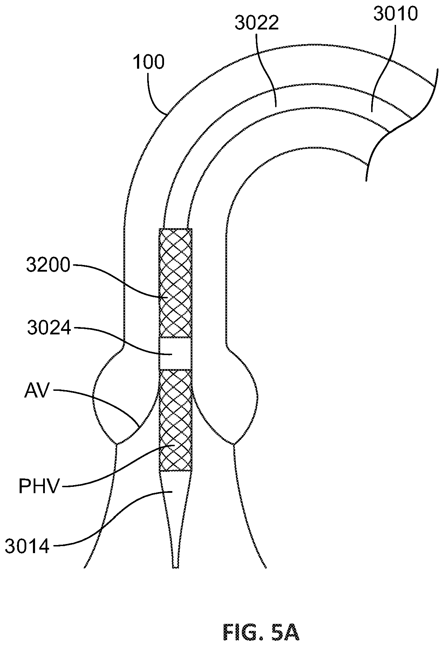

Figs. 5A-C illustrate a delivery device 3010 that includes embolic protection features according to another aspect of the disclosure. Referring to Fig. 5A, a delivery device 3010 is illustrated passing through the aortic arch as a collapsed prosthetic heart valve PHV is being advanced toward the native aortic valve AV. Prosthetic heart valve PHV may be any suitable collapsible and expandable prosthetic aortic heart valve, such as prosthetic heart valve 200. However, the prosthetic heart valve PHV in Figs. 5A-C may have slight construction differences to account for the fact that the prosthetic heart valve PHV is deployed by advancing an overlying sheath, instead of retracting an overlying sheath. For example, the prosthetic heart valve PHV of Figs. 5A-C may have retainers similar to retainers 218 to couple the prosthetic heart valve PHV to the delivery device 3010, but those retainers may be positioned on the outflow end of the device. Delivery device 3010 may be substantially similar or identical to delivery device 1010, with certain modifications described below. However, it should be understood that the modifications may be applied to other prosthetic heart valve delivery devices that are not identical to delivery device 3010.

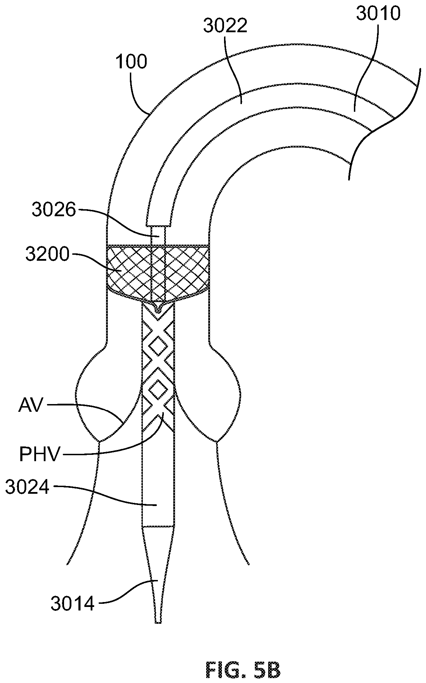

Delivery device 3010 may include an outer sheath 3022 generally similar to outer sheath 1022. Delivery device 3010 may also include a distal sheath or capsule 3024 that is generally similar to distal sheath 1024. However, while delivery device 1010 releases the prosthetic heart valve 200 by retracting the outer sheath 1022 which in turn retracts the distal sheath 1024, the distal sheath 3024 of delivery device 3010 is advanced distally to deploy the prosthetic heart valve PHV. This may be accomplished by any suitable means, for example, including an interior shaft being coupled to the distal tip of 3014 of the delivery device 3010, with the distal tip 3014 being connected or integral with the distal sheath 3024, such that advancement of the inner shaft advances both the distal tip 3014 and the distal sheath 3024, as shown in Figs. 5B-C.

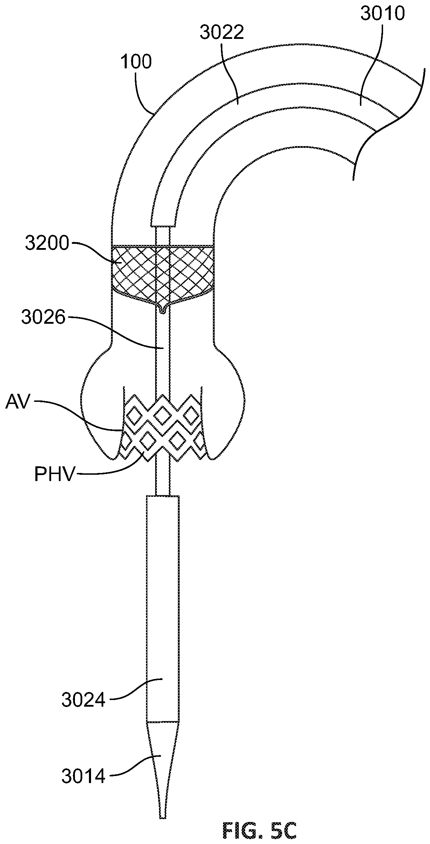

Referring back to Fig. 5A, the distal sheath 3024 may have a length to house not only the prosthetic heart valve PHV in a collapsed condition, but also a filter 3200 in a collapsed condition, the filter being positioned proximal to the prosthetic heart valve PHV in series. The delivery device 3010 may be delivered through the aortic arch and into position within the native aortic valve AV. The inner shaft may be advanced, for example via actuating a wheel or slider on an operating handle outside the patient's body, to begin to advance the distal sheath 3024 distally toward the left ventricle (not labeled). As the distal sheath 3024 advances distally, the overlying constraint on the filter 3200 is released, allowing the filter 3200 to begin to transition from the collapsed condition into the expanded condition, as seen in Fig. 5B. Continued distal advancement of the distal sheath 3024 releases the overlying constraint on the prosthetic heart valve PHV, allowing the prosthetic heart valve PHV to transition into the expanded condition after the filter 3200 has been deployed downstream of the prosthetic heart valve PHV, as shown in Fig. 5C.

Referring still to Fig. 5C, both the filter 3200 and the prosthetic heart valve PHV may overlie a middle shaft 3026. The filter 3200 may include a proximal end, a distal end, and an intermediate portion between the proximal end and the distal end. The distal end of the filter 3200 is preferably attached to the middle shaft 3026. The proximal end of the filter 3200 may be unattached to the middle shaft 3026, and form a circular or tubular shape in the expanded condition, preferably with dimensions such that complete or substantially complete contact with an interior circumference of the aorta 100 is formed. In the illustrated embodiment, the intermediate portion of the filter 3200 includes a substantially conical or frustoconical shape extending from the point of attachment to the middle shaft 3026 toward the proximal end of the filter 3200. It should be understood that, although arteries 102, 104, 106 are not illustrated in Figs. 5A-C, in operation, the arteries 102, 104, 106 would all be positioned downstream of the filter 3200 when the filter 3200 is deployed. The filter 3200 may be formed of any of the materials described above in connection with filter 2200, including a nitinol scaffold with fabric mounted to the scaffold, or braided nitinol, although various other filter materials and structures that allow blood to pass through but inhibit debris from passing through may be suitable. With this configuration, the sequential deployment of filter 3200 prior to the deployment of the prosthetic heart valve PHV may have at least two benefits. First, because the filter 3200 spans across an entire cross-sectional area of a plane of the aorta 100 transverse to the direction of blood flow in the deployed condition, any tissue or other debris entering the flow of blood from the following deployment of the prosthetic heart valve PHV. By trapping the debris within the filter 3200 at a position upstream from the arteries 102, 104, 106 extending from the aorta, the debris is prevented from causing blockages downstream of the filter 3200. Second, the contact of the filter 3200 with the interior wall of the aorta 100 may function to center the middle shaft 3026 within the aorta 100, which may help center the prosthetic heart valve PHV within the center of the annulus of the native aortic valve AV prior to deployment, which is generally desirable. Filter 3200 may include additional features such as folds that create local divots, pockets, or valleys to reduce the likelihood that debris that gets trapped into filter 3200 does not get dislodged or ejected back into the bloodstream as the distal sheath 3024 collapses the filter 3200 back into the collapsed or delivery condition.

After the prosthetic heart valve PHV is in the desired position, as shown in Fig. 5C, the distal sheath 3024 may be retracted by retracting the inner shaft. As the distal sheath 3024 retracts, it passes through the center of the deployed prosthetic heart valve PHV. As the proximal end of the distal sheath 3024 approaches the distal end of the filter 3200, continued retraction of the distal sheath 3024 forces the filter 3200 to collapse back into the distal sheath 3024. This is possible, at least in part, because the distal end of the filter 3200 is in contact with the middle shaft 3026, so that the distal end of the filter 3200 acts as ramp or rail over which the proximal end of the distal sheath 3024 may ride as it retracts. The distal sheath 3024 may be retracted until the proximal end of the distal sheath 3024 abuts the distal end of the outer sheath 3022, at which point the delivery device 3010 may be removed from the patient. As should be understood, there are essentially no additional steps necessary to provide embolic protection in the embodiment of delivery device 3010 compared to the steps that would be required to deliver the prosthetic heart valve PHV without the benefit of a filter that provides embolic protection.

Fig. 6A illustrates a delivery device 4010 that includes embolic protection features according to another aspect of the disclosure. Referring to Fig. 6A, a delivery device 4010 is illustrated passing through the aortic arch as a collapsed prosthetic heart valve PHV is being advanced toward the native aortic valve AV while being maintained in a collapsed condition in the distal sheath 4024. Prosthetic heart valve PHV may be any suitable collapsible and expandable prosthetic aortic heart valve, such as prosthetic heart valve 200. Delivery device 4010 may be substantially similar or identical to delivery device 1010, with certain modifications described below. However, it should be understood that the modifications may be applied to other prosthetic heart valve delivery devices that are not identical to delivery device 4010.

Still referring to Fig. 6A, delivery device 4010 may include a filter 4200a positioned proximal to the distal sheath 4024. Filter 4200a may include a proximal end that is coupled to a stability layer 4090 that may be similar to the stability layers described above, the outer shaft 4022 being translatable with respect to the stability layer 4090. However, it should be understood that the proximal end of the filter 4200a may be directly or indirectly coupled to any component of the delivery device 4010 that is capable of maintaining a substantially static position relative to the aorta 100 while the outer sheath 4022 is retracted for deployment of the prosthetic heart valve PHV. This alternative option of connection of a filter to the stability layer may be true of other embodiment described herein as well. The distal end of filter 4200a may be unattached to the delivery device 4010. Filter 4200a may be formed of any of the structures and/or materials described above for other filters herein, preferably so that blood may flow through the filter 4200a but tissue or other debris are unable to flow through the filter 4200a. Although filter 4200a is illustrated as generally conical or frustoconical, tapering from an open distal end to an attached proximal end, other similar shapes may be suitable.

Fig. 6B is a perspective view of delivery device 4010 with filter 4200a shown in an expanded or deployed state. It should be understood that the filter 4200a shown in Fig. 6A is shown with slight construction differences than the filter 4200a shown in Figs. 6B-D, although the overarching functional concepts are the same. For example, while the filter 4200a shown in Fig. 6A may be formed only of a metal or mesh such as a nitinol wire or braid without a separate fabric or membrane, the filter 4200a shown in Figs. 6B-D includes a metal scaffold or stent that is primarily used as a support for a fabric or membrane filter. As with the other delivery devices described herein, delivery device 4010 may include an operating handle 4020, which may be similar or identical to operating handle 1020 of delivery device 1010.

Fig. 6C is an enlarged view of delivery device 4010 with filter 4200a in an expanded or deployed configuration, with a filter sheath 4300 still overlying a proximal end of filter 4200a. Although filter sheath 4300 may be similar to introducer sheath 1051 of delivery device 1010, the filter sheath 4300 may be a separate overlying sheath specifically for collapsing and deploying the filter 4200a. Although filter 4200a is generally represented as having a conical shape in Fig. 6A, the shape may be other than conical. For example, as shown in Figs. 6B-D, filter 4200a may include a framework of self-expanding materials with a porous fabric extending along and across the framework. Specifically, filter 4200a is shown in Figs. 6B-D as including a plurality of struts, which may be formed from nitinol, with the struts forming a single row of generally diamond-shaped cells 4202a. A fabric 4204a may cover each of the diamond-shaped cells 4202a, such that the fabric 4204a also has a generally diamond-shape for each cell 4202a. However, the fabric 4204a need not exactly follow the shape of the diamond-shaped cells 4202a. For example, the fabric may extend farther distally than is shown in Figs. 6B-D so that the fabric extends between distal peaks of adjacent diamond-shaped cells 4202a. The fabric 4204a may be formed of any suitable material that allows blood to pass through the fabric 4204a, but that does not allow larger particles of debris, such as tissue or emboli, from passing through the fabric 4204a. In one example, fabric 4204a may be a polyurethane material with pores that are laser cut into the material to achieve the desired porosity.

Fig. 6D illustrates the delivery device 4010 after the filter sheath 4300 has been withdrawn further proximally relative to the position shown in Fig. 6C. With the filter sheath 4300 withdrawn further, the stability layer 4090 becomes visible in Fig. 6d. The proximal end of filter 4200a may be coupled to stability layer 4090 by any suitable connection. In the illustrated embodiment, struts at the proximal end of the filter 4200a are clamped or otherwise retained by a crimping member such as a crimp tube 4092. In other embodiments, the proximal end of the filter 4200 may include retainers similar to retainers 218 of stent 202, and complementary cut-outs or recesses may be provided in stability layer 4090 or on a separate member, such as a stainless steel tube positioned on or adjacent the stability layer 4090, with an overlying member positioned radially outward of the recesses to maintain the retainers of the filter in the complementary recesses. Although the distal end of the filter 4200a has a zig-zag shape in the deployed condition as opposed to a continuous circular rim as shown in Fig. 6A, in the deployed condition shown in Figs. 6B-D, the fabric 4202a of the filter may create a complete ring around an inner circumference of the aorta 100 in a plane transverse to the direction of blood flow when the filter 4200a is fully deployed. The material forming the fabric 4204a may be formed onto the framework, for example by dipping or spraying.

Referring again to Fig. 6A, delivery device 4010 may be advanced toward the native aortic valve AV so that the distal sheath 4024, with the prosthetic heart valve PHV maintained in a collapsed condition therein, is positioned within the annulus of the native aortic valve AV. During the delivery, the filter sheath 4300 overlies the filter 4200a so that the filter is maintained in a collapsed condition. The relative spacing between the distal sheath 4024 and the filter 4200a is preferably such that, when the distal sheath 4024 is in the target implant location, withdrawing filter sheath 4300 allows the filter 4200a to expand or otherwise transition to a deployed configuration in which the distal end of the filter 4200a is positioned upstream of the arteries 102, 104, 106 extending from the aorta 100. Thus, prior to deploying the prosthetic heart valve PHV, the filter sheath 4300 is retracted to allow the filter 4200a to transition to the deployed state, for example via self-expansion. With the filter 4200a in place, the outer sheath 4022 may be withdrawn to withdraw the distal sheath 4024 and to deploy the prosthetic heart valve PHV into the native aortic valve AV. As with other embodiments described herein, the outer sheath 4022 may retract into the stability layer 4090 so that the filter 4200a does not change position with respect to the arteries 102, 104, 106 while the distal sheath 4024 is retracted. Due to the shape of the filter 4200a in the expanded or deployed condition, any debris or emboli that dislodge from the deployment of the prosthetic heart valve PHV is likely to enter the interior volume of the filter 4200a and become trapped therein. Once the prosthetic heart valve PHV is in the desired implanted position, the outer sheath 4022 may be advanced to advance the distal sheath 4024 into abutment with the distal tip of the delivery device. Alternatively, the nose cone may be retracted by using a hub similar to hub 1100 of delivery device 1010. Then, the filter sheath 4300 may be advanced relative to the filter to cause the filter to collapse back into the delivery condition, trapping any emboli or debris within the filter 4200a. The delivery device 4010 may then be removed, completing the procedure and removing any trapped debris or emboli from the patient.

Fig. 6E is a highly schematic view of the delivery device 4010 shown in essentially the same position as shown in Fig. 6A, with a first alternate filter 4200b used instead of filter 4200a. The functioning of filter 4200b with respect to delivery device 4010 is essentially identical to the functioning of filter 4200a with respect to delivery device 4010. The main difference is that filter 4200b is formed of a braided material, such as braided nitinol. As with other embodiments disclosed herein, the braid density of filter 4200b may be dense enough so that tissue or other debris cannot pass through the filter 4200b, but not so dense as to prevent blood from passing through the filter 4200b. Alternately, the density of the braid may be small enough to allow for both blood and emboli to pass through, but a fabric or other material may be incorporated into or mounted on the braid to prevent the emboli from passing through the filter 4200b while allowing blood to pass through the filter 4200b. Preferably the filter 4200b is self-expandable and includes a proximal end coupled to the stability layer 4090 and a distal end not directly attached to the delivery device 4010. In the expanded or deployed condition, the distal end of the filter 4200b may contact a continuous inner circumference of the aorta 100 so that blood, and any debris within the flow of blood, must pass through the open distal end of the filter 4200b. The filter 4200b may be generally conical or tubular in the expanded or deployed condition. As with filter 4200a, any emboli trapped within the filter 4200b may remain trapped within the filter 4200b when the filter sheath 4300 is advanced to transition the filter 4200b back into the delivery or collapsed condition.

Fig. 6F is a highly schematic view of the delivery device 4010 shown in essentially the same position as shown in Figs. 6A-B, with a second alternate filter 4200c used instead of filters 4200a or 4200b. The general functioning of filter 4200c with respect to delivery device 4010 is essentially the same to the functioning of filters 4200a and 4200b with respect to delivery device 4010. The main difference is that filter 4200c is formed from a plurality of soft fibers that each have a first end coupled to the stability tube 4090, and extend radially outwardly away from the stability tube 4090 to a second end that is sized to contact the inner wall of the aorta 100. Filter 4200c preferably includes a plurality of fibers positioned along a length of the stability layer 4090, with fibers extending in multiple directions radially away from the stability layer 4090. The density of the fibers of the filter 4200c is preferably high enough to trap tissue or other embolic debris within the fibers of the filter 4200c, while not being so dense as to block blood from flowing through the filter 4200c. Although the fibers of the filter 4200c may be formed from many suitable materials, it is preferable that the material is soft enough so as to reduce risk of puncture or other damage to the inner wall of the aorta 100. On the other hand, the material is preferably stiff enough or has shape-memory properties so as to allow the fibers to be collapsed when the filter sheath 4300 is advanced distally of the filter 4200c, and to allow the fibers of the filter 4200c to spring or otherwise transition outwardly upon retraction of the filter sheath 4300 so as to extend radially outwardly of the stability layer 4090. Materials that may be appropriate for forming the fibers may include polyethylene terephthalate (PET), polyurethane or liquid-crystal polymer (such as Vectran).

Fig. 6G is a highly schematic view of the delivery device 4010 shown in essentially the same position as shown in Figs. 6A-C, with a third alternate filter 4200d used instead of filters 4200a-c. The functioning of filter 4200d with respect to delivery device 4010 is essentially identical to the functioning of filters 4200a-c with respect to delivery device 4010. The main difference is that filter 4200d is formed of a braided material, such as braided nitinol, in the shape of a generally cylindrical disk. As with other embodiments disclosed herein, the braid density of filter 4200d may be dense enough so that tissue or other debris cannot pass through the filter 4200d, but not so dense as to prevent blood from passing through the filter 4200d. Alternately, the density of the braid may be low enough to allow for both blood and emboli to pass through, but a fabric or other material may be incorporated into or mounted on the braid to prevent the emboli from passing through the filter 4200d while allowing blood to pass through the filter 4200d. Preferably the filter 4200d is self-expandable. Rather than forming a conical type of shape with an open interior space, as with filter 4200b, the disk-shaped filter 4200d may have a cable or other connection member 4202d that had a distal end coupled to the filter 4200d, and a proximal end attached to stability layer 4090. In the expanded or deployed condition, the filter 4200d preferably has a size and shape to expand so as to occupy an entire cross-sectional interior area of the aorta 100 in a plane transverse the direction of blood flow. The disk-shaped filter 4200d may have a central aperture to allow outer sheath 4022 to pass through the filter 4200d while the outer sheath 4022 is retracted or advanced, with the central aperture having an inner diameter that is substantially the same or slightly larger than the outer diameter of the outer sheath 4022. In other embodiments, the disk-shaped filter 4200d may be directly coupled over the stability layer 4090 so that the outer sheath 4022 does not ride against an interior surface of the filter 4200d while the outer sheath 4022 is retracted or advanced. Still further, while the illustrated embodiment shows that a proximal end of connection member 4202d is tied, crimped, or otherwise directly attached to stability layer 4090, in other embodiments, the connection member 4202 may be a control wire with a proximal end that extends any desired distance proximally through the delivery device 4090, including to an operating handle. If connection member 4202 is a control wire, it may be possible to retract the filter 4200d into the filter sheath 4300 by pulling the control wire, for example by actuating the operating handle, proximally. As with filters 4200a-c, any emboli trapped within the filter 4200d may remain trapped within the filter 4200d when the filter 4200d is transitioned back into the collapsed or delivery condition within filter sheath 4300.

Fig. 7 illustrates a delivery device 5010 that includes an embolic protection feature according to an aspect of the disclosure. The delivery device 5010 is illustrated passing through the aortic arch as a collapsed prosthetic heart valve (not visible in Fig. 7) is being advanced toward the native aortic valve while being maintained in a collapsed condition by distal sheath 5024. The prosthetic heart valve may be any suitable collapsible and expandable prosthetic aortic heart valve, such as prosthetic heart valve 200. Delivery device 5010 may be substantially similar or identical to delivery device 1010, with certain modifications described below. However, it should be understood that the modifications may be applied to other prosthetic heart valve delivery devices that are not identical to delivery device 1010.

A filter sheath 5300 may be provided overlying the outer sheath 5022, the filter sheath being advanceable and retractable relative to the outer sheath 5022. Preferably, the embolic protection device includes three filters 5200a-c, with each filter 5200a-c being coupled to a distal end of a corresponding control wire 5202a-c, a proximal end of each control wire 5202a-c extending proximally within the filter sheath 5300 to an operating handle that may be similar to operating handle 1020. Each filter 5200a-5200c may be formed of a braided material, such as a collapsible and expandable shape memory material like nitinol. Preferably, each filter 5200a-5200c is disk-shaped or plug-shaped generally similar to filter 4200d. However, each filter 5200a-5200c is preferably sized and shaped such that, in the expanded or deployed condition, each filter 5200a-5200c is able to occupy an entire cross-sectional area of a corresponding artery 102, 104, 106 in a plane transverse the direction of blood flow. As with the other filters described herein, filters 5200a-5200c are preferably configured to allow blood to pass through the filters 5200a-c, while restricting debris and other embolic debris from passing through the filters 5200a-c. Each filter 5200a-5200c may be tethered to a steerable control wire 5202a-5202c so that each filter 5200a-c can be individually steered into a corresponding artery 102, 104, 106.

In an exemplary aortic heart valve replacement procedure, the prosthetic heart valve may be delivered to or near the native aortic valve similar to other methods described herein, with the filters 5200a-c each in a collapsed or delivery condition within the space between filter sheath 5300 and outer sheath 5022. Prior to releasing any of the filters 5200a-c, it is preferable that the distal sheath 5024 be positioned within or adjacent the annulus of the native aortic valve, although in other embodiments the filters 5200a-c may be positioned within the arteries 102, 104, 106 extending from the aorta 100 prior to positioning the distal sheath 5024 within the annulus of the native aortic valve. With the distal sheath 5024 in the desired position, one of the filters 5200a-c may be released from the filter sheath 5300. This may be achieved by retracting the filter sheath 5300 proximal to the filter to be released, using the corresponding control wire to advance the filter distally to the filter sheath 5300, or a combination of both. The steerable control wire may be used, for example by manipulating a corresponding actuator on the operating handle, to guide the released filter into the desired artery. This process may be repeated two more times until each artery 102, 104, 106 has a corresponding filter 5200a-c in position within the artery. At this point, the outer sheath 5022 and distal sheath 5024 may be retracted to transition the prosthetic heart valve into the expanded state within the native aortic valve. Preferably, the retraction of the outer sheath 5022 does not significantly interfere with any of the control wires 5202a-c attached to the filters 5200a-c. This may be achieved, for example, by including control wire lumens within filter sheath 5300 so as to avoid direct contact between the outer sheath 5022 and the control wires 5202a-c. Separate control wire lumens may not be necessary, however, and the control wires 5202a-c may simply remain positioned between the outer shaft 5022 and the filter sheath 5300. If any tissue or embolic debris are dislodged during deployment of the prosthetic heart valve, the embolic debris may become trapped within one of the filters 5200a-c, or otherwise deflected and travel to the downstream aorta where the risk of strike or TIA due to the embolic debris is minimized or eliminated.

After the deployment of the prosthetic heart valve is completed, the filters 5200a-c may be pulled back into the filter sheath 5300 by retracting the control wires 5202a-c. It is expected that the material forming the filters 5200a-c may be soft enough so that little or no damage occurs when pulling the filters 5200a-c out of the corresponding arteries 5200a-c. In order to minimize the distance that each filter 5200a-c needs to travel between the position within the corresponding artery 102, 104, 106 and the collapsed condition within the filter sheath 5300, the distal end of the filter sheath may be advanced to a position near the ostium of the artery in which the first filter to be retrieved is positioned. After the first filter is retrieved back into the filter sheath 5300, the filter sheath 5300 may be repositioned so that its distal end is adjacent the ostium of the artery in which the next filter to be retrieved is positioned. The second filter may be pulled back into the filter sheath using the corresponding control wire. The process may be repeated for the final remaining filter, at which point the delivery device 5010 may be removed from the patient to complete the procedure.

In some aspects, it may be useful to order the delivery and/or retrieval of the filters. For example, during the initial positioning of the filters, it may be preferable to first position the filter 5200a in artery 102, then position filter 5200b in artery 104, and finally position filter 5200c in artery 106. It should be understood that the order of initial placement may be opposite the direction of blood flow. With this initial placement ordering, if placement of the filter dislodges any tissue or other embolic debris, the debris will likely flow in the direction of blood such that any debris dislodged from the first filter 5200a may pass into the descending aorta, any debris dislodged due to the second filter 5200b may be caught or deflected from artery 102 by filter 5200a, and any debris dislodged due to the third filter 5200c may be caught or deflected from arteries 102, 104 by filters 5200a, 5200b. Similarly, it may be preferable to remove the filters 5200a-c in the opposite order after the prosthetic heart valve deployment is complete. The reasoning is substantially similar, in that any debris dislodged by the filter being removed (or dislodged from the filter if the debris is trapped in the filter) will be protected from entering the remaining arteries due to the downstream filters still being in place.

Fig. 8 illustrates a delivery device 6010 that includes an embolic protection feature according to an aspect of the disclosure. The delivery device 6010 is illustrated passing through the aortic arch as a collapsed prosthetic heart valve is being advanced toward the native aortic valve while being maintained in a collapsed condition by a distal sheath (not visible in Fig. 8). The prosthetic heart valve may be any suitable collapsible and expandable prosthetic aortic heart valve, such as prosthetic heart valve 200. Delivery device 6010 may be substantially similar or identical to delivery device 1010, with certain modifications described below. However, it should be understood that the modifications may be applied to other prosthetic heart valve delivery devices that are not identical to delivery device 1010.

A filter sheath 6300 may be provided overlying the outer sheath 6022, the filter sheath being advanceable and retractable relative to the outer sheath 6022. In this embodiment, the embolic protection device includes a fully releasable filter 6200. Filter 6200 may be formed of any suitable material and structure described for the other filters herein. For example, filter 6200 may be formed with a shape-memory metal that forms a lattice or similar framework onto which a fabric or other membrane is positioned so as to allow for blood to flow across the filter 6200 but to prevent tissue or embolic debris from flowing across the filter. Alternately, filter 6200 may be formed from a braided metal, such as a braided shape-memory metal like nitinol. The braid density may be high enough to allow blood, but not embolic debris, to pass through. Otherwise, the braid density may be low but the braid may include a fabric of other membrane within or mounted onto the braid density to provide the desired filtering effect.

Filter 6200 is designed to be fully released from the filter sheath 6300. As a result, filter 6200 preferably has a structure to allow the filter 6200 to maintain a desired position within the aorta 100 and covering the ostia of the arteries 102, 104, 106, while resisting any type of migration from typical forces such as from the flow of blood. One way to achieve this is to have a portion of the filter 6200 have a tubular shape that, upon transition to the expanded or deployed state, creates enough friction with the interior wall of the aorta 100 to prevent accidental movement of the filter 6200. In the illustrated embodiment, the distal end of the filter 6200 has the tubular structure to assist in maintaining the desired position within aorta 100. As is described in greater detail below, the filter 6200 is not to be left in the aorta 100 after completion of the prosthetic heart valve implantation, so it preferably has a mechanism to assist with removal of the filter 6200 after completion of the procedure. In the illustrated embodiment, the structure (*e.g.* the nitinol scaffold or braids) is gathered together at a proximal end of the filter to create a mating feature 6202. It should be understood that the overall shape of filter 6200 in the expanded or deployed condition maintains an interior passageway through the radial center of the filter 6200, so that only a relatively thin layer of filter 6200 is pressed against the interior wall of the aorta 100.

Still referring to Fig. 8, with the prosthetic heart valve and distal sheath positioned in the desired location at or adjacent the annulus of the native heart valve, the filter sheath 6300 may be retracted to fully release the filter 6200 from the space between the filter sheath 6200 and the outer sheath 6022. Preferably, upon release, the filter 6200 covers all of the ostia of the arteries 102, 104, 106 extending from the aorta 100, with the filter 6200 exerting sufficient force radially outward onto the inner wall of the aorta 100 to keep the filter 6200 in the desired position. With the filter 6200 in the desired illustrated position, the prosthetic heart valve may be deployed into the annulus of the native aortic valve, and any tissue or embolic debris that becomes dislodged in the process will be deflected away from the ostia of the arteries 102, 104, 106 by virtue of the filter 6200. With the prosthetic heart valve deployment complete, the outer sheath 6022 may be retracted into the filter sheath 6300.

In order to retrieve the filter 6200, a filter retrieval member 6304 may be provided on an outer surface of a component of the delivery device 6010 that will retract into the filter sheath 6300. In the illustrated example, the retrieval member 6304 may be a magnet, and a corresponding magnet may be provided on the mating feature 6202 at the gathered end of the filter 6200. As the magnetic retrieval member 6304 passes the magnetic mating feature 6202, the magnets will attract and further retraction of the outer sheath 6022 will pull the filter 6200 into the filter sheath 6300. If any embolic debris has been captured within the filter 6200, the embolic debris will also be pulled into the filter sheath 6300, and the delivery device 6010 may be removed from the body to complete the procedure. If the retrieval member 6304 and the mating feature 6202 take the form of magnets, one or both of the magnets may be provided as an electromagnet so that the magnets may be selectively turned on and off to avoid unintentional engagement of the magnets. However, it should be understood that the retrieval member 6304 and the mating feature 6202 may have other suitable corresponding designs to assist in the retrieval of the filter 6300. For example, the mating feature 6202 may form a ring, snare, or a loop, and the retrieval member 6304 may form a hook or grasper to couple to the mating feature. As should be understood, the mating feature 6202 may form the hook or grasper, with the retrieval member 6304 forming the ring, snare, or loop. Any other suitable mating mechanisms may be used to allow the outer sheath 6022 to connect with the filter 6200 and draw the filter 6200 back into the filter sheath 6300 as the outer sheath 6022 is withdrawn into the filter sheath 6300.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the scope of the present disclosure.

## Claims

1. A delivery device (4010) for a collapsible and expandable prosthetic heart valve (200), the delivery device comprising:
an outer sheath (4022) extending from a proximal end to a distal end;
a delivery sheath (4024) positioned at the distal end of the outer sheath, the delivery sheath being sized and shaped to maintain the prosthetic heart valve in a collapsed condition therein; and
a filter sheath (4300) overlying a portion of the outer sheath;
**characterised in that** the delivery device includes an intermediate sheath (4090) positioned between the outer sheath and the filter sheath; and
an embolic filter (4200a, 4200b, 4200c, 4200d) having a proximal end coupled to the intermediate sheath, and a distal end that is not directly attached to the intermediate sheath,
wherein the embolic filter has a delivery condition in which the filter sheath overlies the embolic filter and the distal end of the embolic filter is in a collapsed condition, and a deployed condition in which the filter sheath does not overlie the embolic filter and the distal end of the embolic filter is in an expanded condition.

2. The delivery device (4010) of claim 1, wherein the outer sheath (4022) is translatable with respect to the intermediate sheath (4090).

3. The delivery device (4010) of claim 1, wherein the embolic filter (4200a) includes a structural framework (4202a) and a membrane (4204a) extending across open portions of the framework.

4. The delivery device (4010) of claim 3, wherein the structural framework (4202a) is formed of an expandable shape-memory material.

5. The delivery device (4010) of claim 4, wherein the structural framework (4202a) includes a plurality of struts forming a row of diamond-shaped cells.

6. The delivery device (4010) of claim 5, wherein portions of the plurality of struts at the proximal end of the embolic filter (4200a) are directly fixed to the intermediate sheath (4090).

7. The delivery device (4010) of claim 3, wherein the membrane (4204a) is a porous fabric.

8. The delivery device (4010) of claim 1, wherein the embolic filter (4200b) is formed of a braided mesh.

9. The delivery device (4010) of claim 8, wherein the braided mesh is generally conical or tubular in the deployed condition of the embolic filter (4200b).

10. The delivery device (2010) of claim 1, wherein the distal end of the embolic filter (2210) includes a filter section that has a size and shape in the deployed condition of the embolic filter to span across a plurality of ostia of arteries extending from an ascending aorta.

11. The delivery device (2010) of claim 10, wherein the proximal end of the embolic filter (2210) includes a connection member (2220), a proximal end of the connection member being coupled to the intermediate sheath and a distal end of the connection member being coupled to the filter section.

12. The delivery device (2010) of claim 11, wherein the connection member (2220) and the filter section (2210) are integrally formed.

13. The delivery device (4010) of claim 1, wherein the distal end of the embolic filter (4200d) is generally disk-shaped in the deployed condition of the embolic filter.

14. The delivery device (4010) of claim 13, wherein the proximal end of the embolic filter (4200d) includes a connection member (4202d), a proximal end of the connection member being coupled to the intermediate sheath (4090) and a distal end of the connection member being coupled to the disk-shaped distal end of the embolic filter.

## Patentansprüche

1. Abgabevorrichtung (4010) für eine faltbare und expandierbare Herzklappenprothese (200), wobei die Abgabevorrichtung umfasst:
eine Außenhülle (4022), die sich von einem proximalen Ende zu einem distalen Ende erstreckt;
eine Abgabehülle (4024), die am distalen Ende der Außenhülle positioniert ist, wobei die Abgabehülle eine derartige Größe und Form aufweist, dass die Herzklappenprothese in einem gefalteten Zustand darin bleibt; und
eine Filterhülle (4300), die über einem Teil der Außenhülle liegt;
**dadurch gekennzeichnet, dass** die Abgabevorrichtung eine Zwischenhülle (4090), die zwischen der Außenhülle und der Filterhülle positioniert ist, einschließt; und
einen embolischen Filter (4200a, 4200b, 4200c, 4200d) mit einem proximalen Ende, das mit der Zwischenhülle verbunden ist, und einem distalen Ende, das nicht direkt an der Zwischenhülle befestigt ist, einschließt
wobei der embolische Filter einen Abgabezustand aufweist, in dem die Filterhülle über dem embolischen Filter liegt und das distale Ende des embolischen Filters sich in einem gefalteten Zustand befindet, und einen eingesetzten Zustand aufweist, in dem die Filterhülle nicht über dem embolischen Filter liegt und das distale Ende des embolischen Filter sich in einem expandierten Zustand befindet.

2. Abgabevorrichtung (4010) nach Anspruch 1, wobei die Außenhülle (4022) im Hinblick auf die Zwischenhülle (4090) übertragbar ist.

3. Abgabevorrichtung (4010) nach Anspruch 1, wobei der embolische Filter (4200a) einen strukturellen Rahmen (4202a) und eine Membran (4204a), die sich über offene Teile des Rahmens erstreckt, einschließt.

4. Abgabevorrichtung (4010) nach Anspruch 3, wobei der strukturelle Rahmen (4202a) aus einem expandierbaren Formgedächtnismaterial gebildet ist.

5. Abgabevorrichtung (4010) nach Anspruch 4, wobei der strukturelle Rahmen (4202a) eine Vielzahl von Streben, die eine Reihe von diamantenförmige Zellen bilden, einschließt.

6. Abgabevorrichtung (4010) nach Anspruch 5, wobei Teile der Vielzahl von Streben am proximalen Ende des embolischen Filters (4200a) direkt an der Zwischenhülle (4090) befestigt sind.

7. Abgabevorrichtung (4010) nach Anspruch 3, wobei die Membran (4204a) ein poröses Gewebe ist.

8. Abgabevorrichtung (4010) nach Anspruch 1, wobei der embolische Filter (4200b) aus einem Geflecht gebildet ist.

9. Abgabevorrichtung (4010) nach Anspruch 8, wobei das Geflecht im eingesetzten Zustand des embolischen Filters (4200b) im Allgemeinen konisch oder schlauchförmig ist.

10. Abgabevorrichtung (2010) nach Anspruch 1, wobei das distale Ende des embolischen Filters (2210) einen Filterabschnitt einschließt, der eine derartige Größe und Form im eingesetzten Zustand des embolischen Filters aufweist, dass er sich über eine Vielzahl von Ostien von Arterien, ausgehend von einer aufsteigenden Aorta, erstreckt.

11. Abgabevorrichtung (2010) nach Anspruch 10, wobei das proximale Ende des embolischen Filters (2210) ein Verbindungsglied (2220) einschließt, ein proximales Ende des Verbindungsgliedes mit der Zwischenhülle und ein distales Ende des Verbindungsgliedes mit dem Filterabschnitt verbunden ist.

12. Abgabevorrichtung (2010) nach Anspruch 11, wobei das Verbindungsglied (2220) und der Filterabschnitt (2210) einstückig gebildet sind.

13. Abgabevorrichtung (4010) nach Anspruch 1, wobei das distale Ende des embolischen Filters (4200d) im eingesetzten Zustand des embolischen Filters im Allgemeinen scheibenförmig ist.

14. Abgabevorrichtung (4010) nach Anspruch 13, wobei das proximale Ende des embolischen Filters (4200d) ein Verbindungsglied (4202d) einschließt, ein proximales Ende des Verbindungsgliedes mit der Zwischenhülle (4090) verbunden ist und ein distales Ende des Verbindungsgliedes mit dem scheibenförmigen distalen Ende des embolischen Filters verbunden ist.

## Revendications

1. Dispositif de pose (4010) pour une valve cardiaque prothétique affaissable et expansible (200), le dispositif de pose comprenant :
une gaine externe (4022) s'étendant d'une extrémité proximale à une extrémité distale ;
une gaine de pose (4024) positionnée à l'extrémité distale de la gaine externe, la gaine de pose étant dimensionnée et façonnée pour maintenir la valve prothétique cardiaque dans une condition affaissée dedans ; et
une gaine filtre (4300) recouvrant une partie de la gaine externe ;
**caractérisé en ce que** le dispositif de pose comprend une gaine intermédiaire (4090) positionnée entre la gaine externe et la gaine filtre ; et
un filtre cave (4200a. 4200b, 4200c, 4300d) ayant une extrémité proximale couplée à la gaine intermédiaire, et une extrémité distale qui n'est pas attachée directement à la gaine intermédiaire,
dans lequel le filtre cave a une condition de pose dans laquelle la gaine filtre recouvre le filtre cave et l'extrémité distale du filtre cave est dans une condition affaissée, et une condition déployée dans laquelle la gaine filtre ne recouvre pas le filtre cave et l'extrémité distale du filtre cave est dans une condition dilatée.

2. Dispositif de pose (4010) selon la revendication 1, dans lequel la gaine externe (4022) est déplaçable par rapport à la gaine intermédiaire (4090).

3. Dispositif de pose (4010) selon la revendication 1, dans lequel le filtre cave (4200a) comprend un cadre structurel (4202a) et une membrane (4204a) s'étendant en travers de parties ouvertes du cadre.

4. Dispositif de pose (4010) selon la revendication 3, dans lequel le cadre structurel (4202a) est formé d'un matériau dilatable à mémoire de forme.

5. Dispositif de pose (4010) selon la revendication 4, dans lequel le cadre structurel (4202a) comprend une pluralité d'entretoises formant une rangée d'alvéoles en forme de losange.

6. Dispositif de pose (4010) selon la revendication 5, dans lequel des parties de la pluralité d'entretoises à l'extrémité proximale du filtre cave (4200a) sont fixées directement à la gaine intermédiaire (4090).

7. Dispositif de pose (4010) selon la revendication 3, dans lequel la membrane (4204a) est un tissu poreux.

8. Dispositif de pose (4010) selon la revendication 1, dans lequel le filtre cave (4200b) est formé d'une maille tressée.

9. Dispositif de pose (4010) selon la revendication 8, dans lequel la maille tressée est généralement conique ou tubulaire dans la condition déployée du filtre cave (4200b).

10. Dispositif de pose (2010) selon la revendication 1, dans lequel l'extrémité distale du filtre cave (2210) comprend une section de filtre qui a une taille et une forme dans la condition déployée du filtre cave pour s'étendre en travers d'une pluralité d'ostia s'étendant d'une aorte ascendante.

11. Dispositif de pose (2010) selon la revendication 10, dans lequel l'extrémité proximale du filtre cave (2210) comprend un membre de raccordement (2220), une extrémité proximale du membre de raccordement étant couplée à la gaine intermédiaire et une extrémité distale du membre de raccordement étant couplée à la section de filtre.

12. Dispositif de pose (2010) selon la revendication 11, dans lequel le membre de raccordement (2220) et la section de filtre (2210) sont formés intégralement.

13. Dispositif de pose (4010) selon la revendication 1, dans lequel l'extrémité distale du filtre cave (4200d) est généralement en forme de disque dans la condition déployée du filtre cave.

14. Dispositif de pose (4010) selon la revendication 13, dans lequel l'extrémité proximale du filtre cave (4200d) comprend un membre de raccordement (4202d), une extrémité proximale du membre de raccordement étant couplée à la gaine intermédiaire (4090) et une extrémité distale du membre de raccordement étant couplée à l'extrémité distale en forme de disque du filtre cave.
